# EUROPEAN PATENT APPLICATION

(11) **EP 1 721 970 A1**
(43) Date of publication of application: **15.11.2006**
(21) Application number: 04721016.6
(22) Date of filing: 16.03.2004
(51) Int. Cl.: C12N 15/09

(54) **METHOD OF PREPARING GENOME LIBRARY AND GENOME LIBRARY PREPARED THEREBY**

(30) Priority: 03.03.2004 JP 2004059900
(71) Applicant: Japan as represented by The President of National Institut of Genetics, Mishima-shi Shizuoka 411-8540 (JP)
(72) Inventor: SHIMAMOTO, Nobuo, 4110801 (JP); NAKAYAMA, Hideki, Yata, Mishima-shi, Shizuoka 4110801 (JP); ARAMAKI, Hironori, 8180000 (JP)
(74) Representative: Behnisch, Werner
(86) International application number: PCT/JP2004/003507
(87) International publication number: WO 2005/085434

(57) **Abstract**

There is provided a first method for preparing a genome library in which PCR is carried out with the use of genomic DNA of a target biological organism itself or fragments thereof as a direct template and with the use of one kind of primer with a specified sequence or a random primer so as to effect genome amplification, thereby obtaining a genome library. There is further provided a second method for preparing a genome library in which genome of a target biological organism is pretreated and thereafter PCR is carried out with the use of one kind of primer with a specified sequence so as to effect genome amplification, thereby obtaining a genome library. In both of the methods, a genome library can be prepared conveniently from a small amount of sample.

SEQUENCE LISTING

<110> JAPAN AS REPRESENTED BY THE NATIONAL INSTITUTE OF GENETICS

<120> Genome library, and its production method

<130> U2003P103

<150> JP 2004-59900
<151> 2004-03-03

<160> 20

<170> PatentIn version 3.1

<210> 1
<211> 10
<212> DNA
<213> Escherichia coli

<400> 1
cgcatccggc                                                              10


<210> 2
<211> 10
<212> DNA
<213> Escherichia coli

<400> 2
ccagcgccag                                                              10


<210> 3
<211> 10
<212> DNA
<213> Escherichia coli

<400> 3
gcatccggca                                                              10


<210> 4
<211> 10
<212> DNA
<213> Escherichia coli

<400> 4
gccgcatccg                                                              10


<210> 5
<211> 10
<212> DNA
<213> Escherichia coli

<400> 5

tgccggatgc                                                    10

<210>  6
<211>  10
<212>  DNA
<213>  Escherichia coli

<400>  6
gccggatgcg                                                    10

<210>  7
<211>  10
<212>  DNA
<213>  Escherichia coli

<400>  7
cagcgccagc                                                    10

<210>  8
<211>  10
<212>  DNA
<213>  Escherichia coli

<400>  8
ctggcgctgg                                                    10

<210>  9
<211>  10
<212>  DNA
<213>  Escherichia coli

<400>  9
gcctgatgcg                                                    10

<210>  10
<211>  10
<212>  DNA
<213>  Escherichia coli

<400>  10
cgccgcatcc                                                    10

<210>  11
<211>  10
<212>  DNA
<213>  Escherichia coli

<400>  11
gctggcgctg                                                    10

<210> 12
<211> 10
<212> DNA
<213> Escherichia coli

<400> 12
cggataaggc 10

<210> 13
<211> 10
<212> DNA
<213> Escherichia coli

<400> 13
tgcctgatgc 10

<210> 14
<211> 10
<212> DNA
<213> Escherichia coli

<400> 14
acgccgcatc 10

<210> 15
<211> 10
<212> DNA
<213> Escherichia coli

<400> 15
cggatgcggc 10

<210> 16
<211> 10
<212> DNA
<213> Escherichia coli

<400> 16
ggatgcggcg 10

<210> 17
<211> 10
<212> DNA
<213> Escherichia coli

<400> 17
ggcgctggcg 10

<210> 18
<211> 10
<212> DNA

<213>  Escherichia coli

<400>  18
ccgcatccgg                                                                10

<210>  19
<211>  10
<212>  DNA
<213>  Escherichia coli

<400>  19
cgccagcgcc                                                                10

<210>  20
<211>  10
<212>  DNA
<213>  Escherichia coli

<400>  20
ggataaggcg                                                                10

## Description

### Technical field

The present invention relates to a simple and an easily operable method for preparing a genome library from a very small amount of samples and the genome library prepared by the method. The present invention can be widely applied as a study material for genome analyses of various organisms, the development of genome-based medicines, and studies of other fields of life science.

### Background of the invention

Conventional methods for preparing genome libraries have been carried out by a shot gun cloning method and cloning of cut genome fragments into plasmids, cosmids, phages, artificial chromosome vectors and others.

However, the conventional methods mentioned above require many treatments such as a cutting process with a restriction enzyme and integration of genome fragments into vectors. These methods require a large amount of genomic DNAs as sample materials and accompanied troublesome processes. Therefore, the preparation of a genome library was difficult, particularly for microorganisms having difficulties in their culture.

On the other hand, the method for preparing a genome library using the PCR (polymerase chain reaction) was reported by Singer et al., in 1997 [Nucleic Acids Research, 25(4) 781-786 (1997)]. However, the method also requires fragmentation using a restriction enzyme, followed by a linker connection process to each cut genome fragment and other processes. Thus, the method required troublesome pretreatment steps before carrying out the PCR, the loss of genomic DNAs accompanied by these steps was unavoidable, and it was difficult to easily prepare a genome library from a very small amount of samples. Furthermore, the method used plural kinds of primers in the PCR.

It goes without saying that analytical studies at a genome level in the fields of biology and life science are important, and the analytical studies for various organisms at their genome levels practically became important. For example, the analytical studies for not only pathogenic bacteria and viruses but also unknown or little known microorganisms and viruses living around the environment will become more important in view of public health and safety. An easily operable method for preparing their genome libraries from a very small amount of samples is demanded for these analytical studies.

### Disclosure of the invention

The object of the present invention is to solve the above-mentioned problems and provide an easily operable method for preparing a genome library from a very small amount of samples and the genome library prepared by the method.

The inventors found that the PCR using one kind of primer designed on the basis of comparatively commonly appearing sequence within a target genome almost uniformly amplifies whole genome area and a genome library can be easily prepared, etc. These findings led us to the present invention.

That is the present invention includes the below-mentioned inventions of A) to M) as industrially useful inventions.
A) A method for preparing a genome library of any biological organisms, characterized by use of a PCR to amplify a genome, the PCR using as a template a genomic DNA of a target organism or its fragments, and using one kind of primer with a specific sequence or a random primer.
B) A method for preparing a genome library according to the above A), characterized by use of an oligo-DNA as a primer designed so as to include a frequently appearing sequence within a genome of a target organism.
C) A method for preparing a genome library according to the above B), characterized by use of an oligo-DNA as a primer designed so as to include a frequently appearing sequence of 6mer or more.
D) A method for preparing a genome library according to the above B) or C), characterized by use of an oligo-DNA as a primer designed so as to have a frequently appearing sequence at its 3'-terminal side, and further to have, at its 5'-terminal side, a sequence with no or low frequency within a genome of a target organism.
E) A method for preparing a genome library according to any of the above B) to D), characterized by use of an oligo-DNA as a primer designed so as to have a frequently appearing sequence at its 3'-terminal side, and further to have an area of 1mer or more composed of a random base and/or a universal base at the 3'-terminal side of the frequently appearing sequence.
F) A method for preparing a genome library according to the above A), characterized by use of an oligo-DNA as a primer designed so as to have an area of 6mer or more composed of a random base and/or a universal base at its 3'-terminal side.
G) A method for preparing a genome library according to the above F), characterized by use of an oligo-DNA as a primer designed so as to have an area of 6mer or more composed of a random base and/or a universal base at its 3'-terminal side, and further to have, at its 5'-terminal side, a sequence with no or low frequency within a genome of a target organism.
H) A method for preparing a genome library, characterized by carrying out a first PCR using the primer according to the above D) or G), and followed by a second PCR using a primer including the 5'-terminal side sequence.
I) A method for preparing a genome library according to any of the above A) to H), characterized by a PCR condition having a cycle with an annealing temperature set within the range of 30-45°C, and a temperature raising period set within the range of 5 seconds to 20 minutes, said temperature raising period being time for shifting from the annealing temperature to an extension reaction temperature.
J) A method for preparing a genome library of any biological organisms, characterized by carrying out a pretreatment to a genome of a target organism, and followed by a PCR to amplify a genome, the PCR using one kind of primer with a specific sequence.
K) A method for preparing a genome library according to the above J), characterized by the pretreatment in which the oligo-DNA according to any of the above B) to G) is used as a primer to amplify a genome, and followed by the PCR using one kind of primer with a specific sequence, to amplify a genome again.
L) A method for preparing a genome library according to the above J), characterized by the pretreatment in which a genome is fragmented and an additional sequence such as a linker is connected to each fragment, and followed by the PCR using one kind of primer with a specific sequence, to amplify a genome.
M) A genome library prepared by the method according to any of the above-mentioned A) to L).

The first method of the present invention prepares a genome library by carrying out the PCR using a genomic DNA itself or its fragments of a target organism as a direct template. That is, the genomic DNA itself without any additional sequence other than the genome sequence such as a linker is used as the template for the PCR. Therefore, any troublesome PCR pretreatments, such as cutting of extracted genomic DNAs with a restriction enzyme and connection of a linker to the resultant each fragment are not required and the loss of samples accompanied to these pretreatments can be prevented.

In addition, a genome library can be prepared by carrying out the PCR using bacterial or cultured cells as a direct sample according to the first method of the present invention. In this case, no extraction process of a genomic DNA is required and a genome library can be more easily prepared.

The second method of the present invention simply and easily prepares a genome library by carrying out a pretreatment to a genomic DNA of a target organism, followed by the PCR using one kind of primer with a specific sequence. A genome library can be simply and easily prepared by the PCR using only one kind of primer.

As described above, a genome library can be simply and easily prepared according to the method of the present invention from a very small amount of samples.

Further other purposes, characteristic features and advantages will be sufficiently understood by the following descriptions. The benefits of the present invention will become evident by the following explanations referring to the attached drawings.

### Brief description of the drawings

Figure 1 is a drawing explaining the outline of a method for preparing a genome library of the present invention.
Figure 2 is a drawing explaining the amplification of a genome by PCR using primers different from each other, and the results examined by a gel electrophoresis, in a genome library production method of one example of the present invention.
Figure 3 is a drawing showing the amplification of a genome by PCR using a primer 2 under conditions different from each other, and the results examined by a gel electrophoresis, in a genome library production method of one example of the present invention.
Figure 4 is a drawing explaining various primers used in the experiments to confirm whether whole genome area is practically amplified or not, in the genome library prepared by one example of the present invention, and each genome area amplified by these primers.
Figure 5 is a drawing explaining results of the experiments to confirm whether whole genome area is practically amplified or not, in the genome library prepared by one example of the present invention.
Figure 6 is a graph explaining results of comparison between the sequence on primer 2 used by one example of the present invention and sequences on a genome.

### The best mode for carrying out the invention

The practical embodiments of the present invention will be explained in detail. (1) The first method for preparing a genome library

The first method for preparing a genome library of the present invention prepares a genome library by carrying out a PCR using as a template a genomic DNA itself or its fragments, without any additional sequence other than the genome sequence such as a linker. The PCR using a genomic DNA as the template includes not only the use of extracted genomic DNA as a template but also the direct use of bacterial or cultured cells without extraction of genomic DNA, and the PCR using such an intrabacterial or intracellular genomic DNA as a template.

Here, the characteristic feature of the primer of the present invention will be explained. The primers used in the PCR of the present invention are roughly classified into (1) one kind of primer with a specific sequence or (2) a random primer containing a random sequence.

Among them, an oligo-DNA designed so as to include a sequence with high frequency in a target genome is preferably used in case when one kind of primer with a specific sequence is used as a primer. The PCR in the present invention is a random PCR which randomly amplifies the whole area of the genome. In other words, the PCR is required to amplify the whole area of genome as uniformly as possible. Thus, the primer non-specifically annealing (complimentarily binding) plural areas on a target genome is preferred.

Furthermore, as will be shown in the later examples, the inventors found the importance of 6mer base sequence as an annealing area of the primer and thus, the use of an oligo-DNA designed so as to include a frequently appearing sequence of 6mer or more is preferable.

A frequently appearing sequence in a target genome can be determined according to available sequence information of a target genome. In this case, it is not required that all the genome sequences are known. The frequently appearing sequence may be determined on the basis of a partially known genome sequence. Or, the frequently appearing sequence may be determined from the resultant sequence information obtained by a random sequencing of a target genome prior to the PCR of the present invention.

For example, when a primer is designed so as to contain frequently appearing 6mer sequence, frequently appearing sequences may be orderly determined from the highest frequency in all the 6mer sequences included in sequence information of a target genome. Then, any sequence may be selected among frequently appearing 1st to 20th sequences and the primer may be designed to include the sequence. As mentioned above, "frequently appearing sequence" is not restricted to the sequence with the highest frequency and any sequence may be selected among the sequences with a comparatively high frequency.

The length of primers used in the present invention is not particularly limited, but a shorter primer in comparison to those used in the conventional PCR is preferable, and those having 8mer to 15mer is practically desirable in considerations of annealing temperature and random annealing on a target genome.

For example, when a primer of 15mer length is designed, such primer may be designed so as to include a frequently appearing 6mer to 15mer sequence. In the Examples described later, the length of a primer was set as 10mer, and the primer was designed to include a frequently appearing 10mer sequence and used for the PCR. In the Examples, the whole genome area was successfully and almost uniformly amplified. Thus, a primer may be preferably designed to have the length of 10-12mer and to include a frequently appearing 6-12mer sequence, for example.

In addition, it is preferable to design a primer to have a frequently appearing sequence at its 3'-terminal side.

One or plural bases may be universal bases or random bases (random sequences) in the frequently appearing sequence included in the designed primer. The universal base is a base capable to form a hydrogen bond with each base of adenine (A), thymine (T), guanine (G) or cytosine (C), and the 5-nitroindole and the 3-nitropyrrole are illustrated. The random base (random sequence) is a base randomly synthesized to any one of A, T, G and C at a specific site in the sequence. Primers (random primers) including a random base (a random sequence) may be synthesized in one step synthesis by addition of almost equal amounts of A, T, G and C as a base at specific site. Thus, a primer can be easily synthesized by one step synthesis, even when using a random primer in the present invention.

As mentioned above, a primer used in the present invention may be preferably designed so as to have a frequently appearing sequence at its 3'-terminal side. Additionally, a primer may be designed so as to have an area of 1mer or more composed of a random base (a random sequence) or a universal base at the 3'-terminal side of the frequently appearing sequence.

Furthermore, a primer may be designed to have an area of 6mer or more composed of a random base (a random sequence) or a universal base at its 3'-terminal side without a frequently appearing sequence. As described above, the results of the analysis carried out by the inventors found the importance of 6mer base sequence as an annealing area in a used primer. By having an area of 6mer or more composed of a random base (a random sequence) or a universal base, a primer may anneal non-specifically on the genome.

It is preferable to design a primer so as to have a frequently appearing sequence at its 3'-terminal side, and further to have, at its 5'-terminal side, a sequence with no or low frequency within a genome of a target organism. For example, such primer may be designed to have a frequently appearing sequence of 6mer or more at its 3'-terminal side, together with a 6mer or more sequence with no or low frequency in a target genome at its 5'-terminal side. Designing of such primer allows to carry out the first PCR using the said primer, followed by the second PCR using a primer having the above-mentioned 6mer or more sequence at the 5'-terminal side, to give a more stable amplification of each amplified genome fragment.

Similarly, it is also preferable to design a primer so as to have a specific sequence with no or low frequency in a target genome at the 5'-terminal side, in case (1) when a primer is designed to have a frequently appearing sequence at its 3'-terminal side and further to have an area of 1mer or more composed of a random sequence or a universal base at the 3'-terminal side of the frequently appearing sequence, and also in case (2) when a primer is designed to have an area of 6mer or more composed of a random sequence or a universal base at its 3'-terminal side. For example, designing of such primer may be carried out to have an area of 6mer or more composed of a random sequence or a universal base at its 3'-terminal side, together with a 6mer or more sequence with no or low frequency in a target genome at its 5'-terminal side. Designing of such primer also provides a more stable amplification of each amplified genome fragment by carrying out the first PCR using the said primer, followed by the second PCR using a primer having the above-mentioned 6mer or more sequence at the 5'-terminal side.

Here, the meaning of "a sequence with low frequency" is not restricted to a sequence with the lowest frequency. Similarly to the meaning of "a frequently appearing sequence", any sequence may be selected among plural sequences with comparatively low frequency.

In the present invention, "a random PCR" is carried out using any primer described above, to give random and nonspecific amplifications of a target genome, and it is different from the conventional PCR amplifying a specific area on the genome sandwiched between two primers.

The PCR conditions used in the present invention will be explained.

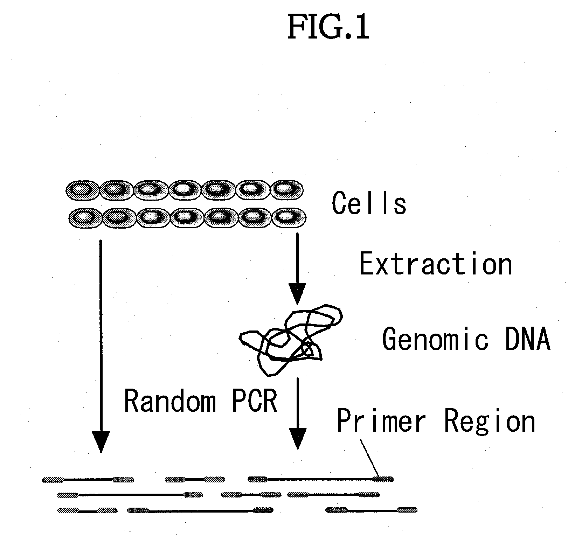

At first, a genomic DNA is extracted with an alkaline, a phenol, a guanidine or available kits, prior to the PCR where the extracted genomic DNA is used as a template. The extracted genomic DNA may be fragmented with a restriction enzyme, however, the extracted genomic DNA itself may be used as a template in the present invention. Of course, no complicated pretreatment such as to give a linker to the genomic DNA is required prior to the PCR. In addition, as explained earlier, the PCR may be carried out using bacterial and cultured cells directly as a sample without extraction of the genomic DNA and intrabacterial or intracellular genomic DNA as a template (see Figure 1).

In the PCR conditions, an annealing temperature is preferably set at a low temperature for effective annealing of a used short primer to whole genome area, followed by gradual elevation of the temperature up to an extension reaction temperature, to prevent separation of an annealed primer at the extension reaction temperature, so that extension reaction from an annealed primer may be carried out at a slow rate.

Therefore, the PCR conditions preferably have a cycle with an annealing temperature set within the range of 30-45°C, a temperature raising period set within the range of 5 seconds to 20 minutes, and an extension reaction time set within the range of zero second to 15 minutes. The temperature raising period is time for shifting from the annealing temperature to the extension reaction temperature. Such cycle (of low annealing temperature, and gradual elevation for slow extension reaction) may be adopted only in initial several cycles, but alternatively such cycle may be adopted throughout all the cycles. ("zero second" for the extension reaction time is when temperature is set to keep elevation from the annealing temperature to a denaturing temperature via the extension reaction temperature, without staying at the extension reaction temperature.)

The PCR conditions, other than those described above, may be determined according to known methods, such as the number of cycles, a denaturing temperature to give a single stranded DNA, composition of PCR buffer, and a used DNA polymerase, without any restriction.

As described in the later examples, the random PCR according to the above-mentioned conditions results in an almost uniform amplification of whole genome area and an easily operable preparation of a genome library is confirmed. In addition, the method of the present invention requires no complicated treatment such as a linker addition to the genome or integration of genome fragments into the vector, and a genome library can be easily prepared from a very small amount of samples without accompanying the loss of samples.

The method for preparing a genome library of the present invention and the prepared genome library by the method can be widely applied to study materials for the genome analysis of various organisms, development of genome-based medicines, and various other field studies of biological science. For example, the present invention can be applied for the analyses and the studies of unknown or little known environmental microorganisms or viruses. Particularly, the analyses and the studies of pathogenic bacteria and viruses are important in view of public hygiene and establishment of national health, and the present invention is useful for these analyses and studies.

Biological organisms capable to prepare genome libraries by the present invention have no particular restriction and include, for example, plants and animals in addition to microorganisms and viruses, and of course may be mammals including human beings. A genome library for viral RNA genomes may be prepared by the synthesis of a complementary stranded DNA with a reverse transcriptase and use of the DNA as a template.

As described above, the present invention simply and easily prepare a genome library by the PCR using one kind of primer with a specific sequence or a random primer. The use of one kind of primer or a random primer is preferable in view of simplicity and cost, however, the present invention is not restricted to the use of one kind of primer or a random primer. For example, two primers respectively with the first and the second frequently appearing sequences may be used for the PCR. That is, the present invention may be a method carrying out the PCR using at least one kind of primer or a random primer.

A 6mer base sequence is important for an annealing area of a used primer as described earlier, however, as shown in the later examples, complementation of at least five bases within the 6mer is satisfactory to non-specific amplification by the PCR. Thus, one base within the 6mer may be different from a genome sequence by. (2) The second method for preparing a genome library

The second method for preparing a genome library of the present invention is performed by pretreatment to the genome of a target biological organism, and followed by a PCR using a primer with one specific sequence for amplification of the genome. Random primer is not included in the "primer with one specific sequence".

Thus, the above method of the present invention prepares a genome library by a PCR using only one kind of primer and a simply operable preparation of a library can be realized.

The following pretreatments may be illustrated as the pretreatment of the PCR.
i) As the pretreatment, a PCR is performed according to the aforementioned conditions, using "an oligo-DNA designed so as to have a specific sequence with no or low frequency in a target genome at its 5'-terminal side" among the aforementioned primers. The 3'-termnal side of the said primer may have s frequently appearing sequence of 6mer or more, or a sequence of 6mer or more composed of a random base (a random sequence) or a universal base, as described above. For example, a primer with a random sequence at its 3'-terminal side and a specific sequence at its 5'-terminal side is used, and the PCR is carried out as the pretreatment, using this primer composed of the random sequence and the specific sequence.

Then, the second PCR is carried out using one kind of primer with the above specific sequence, to prepare a genome library by re-amplification of a target genome.

In the pretreatment PCR, a 6mer base sequence at the 3'-terminal side of a used primer is important for an annealing with a target genome. Any sequence may be adopted as a specific sequence at the 5'-terminal side. In the second PCR, a primer having an additional sequence at the 5'-terminal side of this specific sequence may be used.
ii) Other pretreatments include fragmentation of a genome with a restriction enzyme or a physical cutting, and connection of an additional sequence such as a linker to each fragment. The additional sequential part is made as an annealing area of a used primer and in the following PCR, a genome is amplified using one kind of primer with a specific sequence corresponding to the above additional sequence, to prepare a genome library.

As described above, connection of a determined specific sequence to each genome fragment is carried out as the pretreatment, followed by the PCR using one kind of primer with the above specific sequence (or, a sequence having an additional sequence at the 5'-terminal side of the above specific sequence) to prepare a genome library.

After the above mentioned i) or ii) pretreatment, the PCR may be performed using one kind of primer with a specific sequence under the aforementioned PCR conditions, however, higher temperatures may be used for an annealing, and other conditions may be changed.

In addition, the length of a primer used in the above-mentioned PCR is not particularly limited but a shorter primer than those used in the conventional PCR is preferable and the 8-15mer length is preferable in view of annealing temperature and random annealing on a target genome.

Hereinafter, the examples of the present invention will be explained referring to the drawings, however, the present invention is not restricted by these examples.

### [Example 1]

The method for preparing a genome library of the present invention may be carried out by extraction and purification of a genomic DNA followed by the PCR, or by the PCR using cells and bacterial cells as a direct sample, as shown in Fig. 1. In the present example, the PCR was carried out using an extracted genomic DNA as a template.

In the present example, a *Escherichia coli* genome library was prepared through amplification of a genomic DNA from *Escherichia coli* K-12 W3110 strain by a random PCR.

The primer used in the random PCR was designed on the basis of 10mer sequences with high frequency in a target genome. More specifically, the 10mer sequences with high frequency on the genome of *E. coli* K-12 W3110 were searched. In the whole genome length of 4,639,221 bases, the number of 10mer sequences was 4,639,212 bases in total, and sequences with high frequency from the 1st to the 20th were extracted and determined. The results are shown in Table 1.

**[Table 1]**

| | | a | b |
|---|---|---|---|
| 1. | CGCATCCGGC | 149 | 0.0032 |
| 2. | CCAGCGCCAG | 143 | 0.0031 |
| 3. | GCATCCGGCA | 142 | 0.0031 |
| 4. | GCCGCATCCG | 136 | 0.0029 |
| 5. | TGCCGGATGC | 136 | 0.0029 |
| 6. | GCCGGATGCG | 134 | 0.0029 |
| 7. | CAGCGCCAGC | 134 | 0.0029 |
| 8. | CTGGCGCTGG | 125 | 0.0027 |
| 9. | GCCTGATGCG | 124 | 0.0027 |
| 10. | CGCCGCATCC | 122 | 0.0026 |
| 11. | GCTGGCGCTG | 120 | 0.0026 |
| 12. | CGGATAAGGC | 115 | 0.0025 |
| 13. | TGCCTGATGC | 114 | 0.0025 |
| 14. | ACGCCGCATC | 113 | 0.0024 |
| 15. | CGGATGCGGC | 112 | 0.0024 |
| 16. | GGATGCGGCG | 111 | 0.0024 |
| 17. | GGCGCTGGCG | 109 | 0.0023 |
| 18. | CCGCATCCGG | 109 | 0.0023 |
| 19. | CGCCAGCGCC | 108 | 0.0023 |
| 20. | GGATAAGGCG | 107 | 0.0023 |
| 4,639,212 fragments in 4,639,221 residues | | | |
| a: Number of appearance | | b: Ratios to whole 10mer | |

| | | | |
|---|---|---|---|
| The 10mer sequences listed in the above Table 1 are also shown in SEQ Nos. 1-20 in the sequence listing. | | | |

The primers used in the random PCR were synthesized primers respectively with the above No.1 to No.4 sequences among the above 10mer sequences (hereinafter referred as primers 1-4, respectively).

The composition of the PCR solution was as follows:

| | |
|---|---|
| Template: Genomic DNA (Extracted by a conventional method after proliferation of *E.coli*) | 80 ng/mL |
| Primer: One kind of primer from primers 1-4 | 20 *µ*M |
| dNTP | 0.2 mM |
| DNA polymerase: | TaKaRa Z-taq™ 0.2 U |
| Buffer: | × 10 PCR buffer 1 *µ*L |

Diluted up to 10 *µ*L with dH₂O

The PCR conditions were firstly denaturing at 98°C for five seconds and then 40 times cycles shown below.
Denaturing temperature: 98°C for five seconds
Annealing temperature: 42°C for 10 seconds
Inclination of temperature changes from the annealing temperature to the extension reaction temperature (RAMP): 5% (five minutes) Further, the set values were variously changed and the PCR was performed.
Extension reaction temperature: 72°C for seven minutes

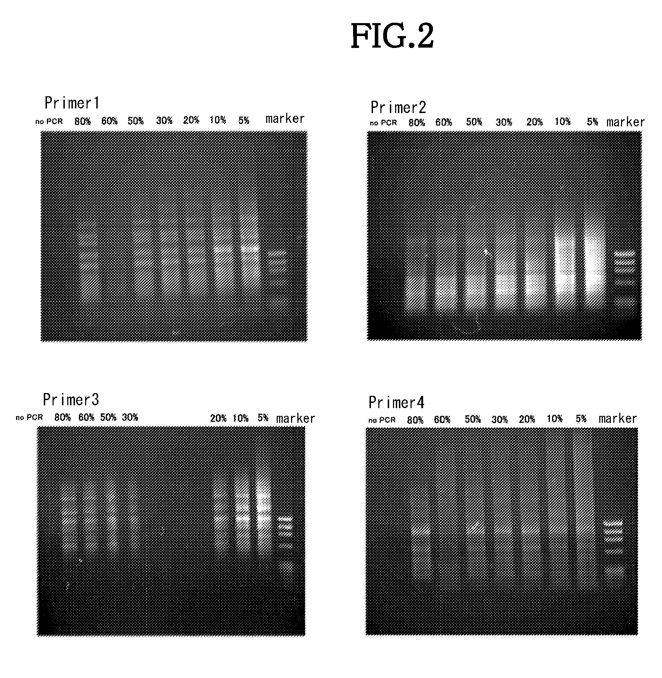

The PCR was carried out according to the above-mentioned conditions and the results were confirmed with an electrophoresis after amplification. The results showed specific amplification bands for the primer 1, 3 and 4 as shown in Fig. 2, however, the primer 2 showed a smear for the amplified product, which suggested amplification throughout the whole genome area. In Fig. 2, percentages (%) represent the set values for the above RAMP, and the higher the percent value the sooner temperature ramp time from annealing to extension reaction, while the smaller the percent value the longer period is required to raise the temperature.

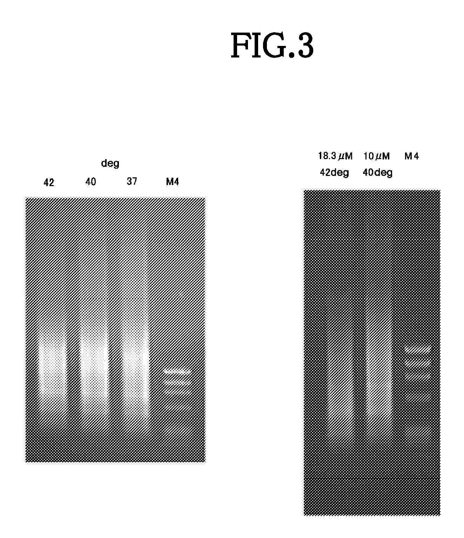

Further, similar PCR reactions were carried out using the above primer 2 under various primer concentrations. As a result, the lower the primer concentration, the more decrease of specific amplification was observed, and the amplification of the whole genome into short fragments was confirmed (see Fig. 3). The PCR reactions were also performed under various annealing temperatures ("deg" in the Fig. 3) and optimization of the temperature and the primer concentration gave more smear-like results in electrophoresis, and the amplification throughout the whole genome was confirmed.

The similar amplification was repeated using the amplified genome fragments as a template. As a result, amplification of long DNA fragments decreased. Further repetition of the amplification experiment showed further decrease of amplified long DNA fragments; however, their decrease changes became less.

### [Example 2]

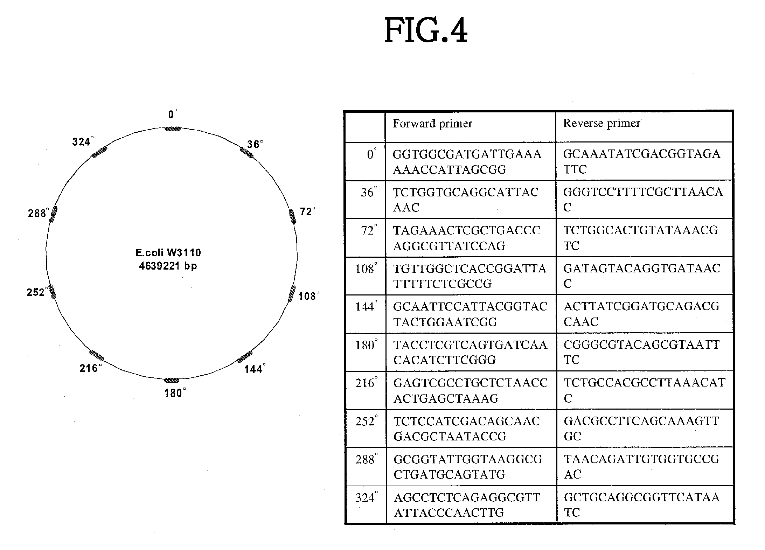

Determination of density ratios for 10 points on the genome of *E. coli* K-12 W3110 strain was carried out to confirm whether the whole genome area is practically amplified or not for the genome library prepared in Example 1. The determination points are shown in Figure 4. The determination of density ratios was performed with a LightCycler™ (Roche Diagnostic K.K.) for the genome fragments amplified by the random PCR. Primers (the forward primer and the reverse primer) used for the determination at each determination point (area) are also shown in Figure 4.

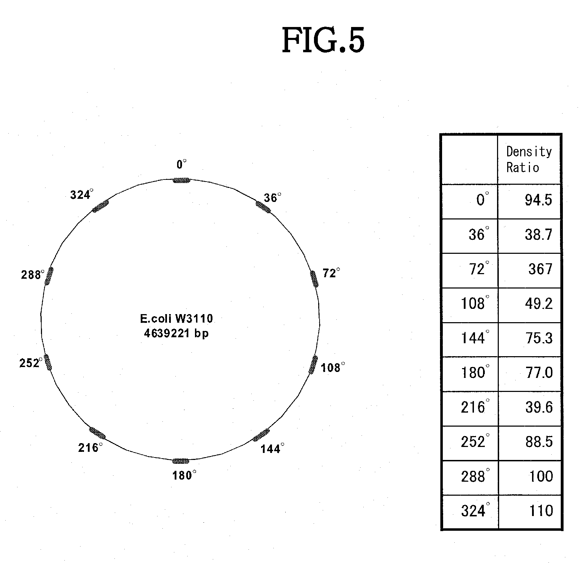

The results of the above determinations are shown in Figure 5. The ratio of density between areas (in other words, ratio of amplification) was within 10-fold, even in the largest difference. The results showed almost uniform amplification throughout the whole genome area in the prepared genome library.

### [Example 3]

A part of the genome library prepared in Example 1 was subjected to sequence analysis. After the sequence analysis, the results were compared with those of known genome sequences using BLAST (Basic Local Alignment Search Tool). The results showed amplification throughout the whole genome area.

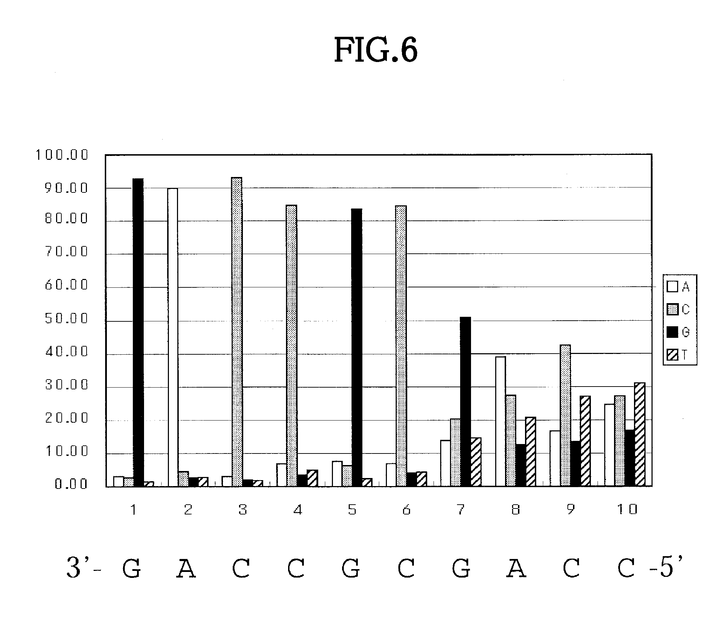

Furthermore, the sequence of the above primer 2 used in the PCR of the present Example was compared with sequences on the genome, on the basis of amplified fragments. The results confirmed a very high consistency with sequences on the genome up to six bases (6mer) at the 3'-terminal side of the primer sequence as shown in Figure 6 (in the Figure, the vertical axis shows percentage). Therefore, the 6mer base sequence is very important as an annealing area of the primer.

Dispersion of the base sequences at the above six bases was investigated. As a result, amplification was confirmed, even if one base was different.

As described above, the primer used for preparing a genome library is preferably designed so that a base sequence of six bases at its 3'-terminal side coincide with a frequently appearing sequence in a target genome. Or, the primer is preferably designed so that a base sequence of at least five out of six bases at its 3'-terminal side coincide with a frequently appearing sequence in a target genome.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

### Industrial applicability

As described above, a genome library can be simply and easily prepared from a very small amount of samples by the present invention and is applicable to studies and analyses of pathogenic bacteria and viruses, and unknown or little known environmental microorganisms and viruses. Furthermore, the present invention can be widely used for the study materials of genomic analyses for various organisms, and for the development of genome-based medicines and studies of various life science fields.

## Claims

1. A method for preparing a genome library of any biological organisms, **characterized by** use of a PCR to amplify a genome, the PCR using as a template a genomic DNA of a target organism or its fragments, and using one kind of primer with a specific sequence or a random primer.

2. A method for preparing a genome library according to claim 1, **characterized by** use of an oligo-DNA as a primer designed so as to include a frequently appearing sequence within a genome of a target organism.

3. A method for preparing a genome library according to claim 2, **characterized by** use of an oligo-DNA as a primer designed so as to include a frequently appearing sequence of 6mer or more.

4. A method for preparing a genome library according to claim 2 or 3, **characterized by** use of an oligo-DNA as a primer designed so as to have a frequently appearing sequence at its 3'-terminal side, and further to have, at its 5'-terminal side, a sequence with no or low frequency within a genome of a target organism.

5. A method for preparing a genome library according to any of claims 2-4, **characterized by** use of an oligo-DNA as a primer designed so as to have a frequently appearing sequence at its 3'-terminal side, and further to have an area of 1mer or more composed of a random base and/or a universal base at the 3'-terminal side of the frequently appearing sequence.

6. A method for preparing a genome library according to claim 1, **characterized by** use of an oligo-DNA as a primer designed so as to have an area of 6mer or more composed of a random base and/or a universal base at its 3'-terminal side.

7. A method for preparing a genome library according to claim 6, **characterized by** use of an oligo-DNA as a primer designed so as to have an area of 6mer or more composed of a random base and/or a universal base at its 3'-terminal side, and further to have, at its 5'-terminal side, a sequence with no or low frequency within a genome of a target organism.

8. A method for preparing a genome library, **characterized by** carrying out a first PCR using the primer according to claim 4 or 7, and followed by a second PCR using a primer including the 5'-terminal side sequence.

9. A method for preparing a genome library according to any of claims 1-8, **characterized by** a PCR condition having a cycle with an annealing temperature set within the range of 30-45°C, and a temperature raising period set within the range of 5 seconds to 20 minutes, said temperature raising period being time for shifting from the annealing temperature to an extension reaction temperature.

10. A method for preparing a genome library of any biological organisms, **characterized by** carrying out a pretreatment to a genome of a target organism, and followed by a PCR to amplify a genome, the PCR using one kind of primer with a specific sequence.

11. A method for preparing a genome library according to claim 10, **characterized by** the pretreatment in which the oligo-DNA according to any of claims 2-7 is used as a primer to amplify a genome, and followed by the PCR using one kind of primer with a specific sequence, to amplify a genome again.

12. A method for preparing a genome library according to claim 10, **characterized by** the pretreatment in which a genome is fragmented and an additional sequence such as a linker is connected to each fragment, and followed by the PCR using one kind of primer with a specific sequence, to amplify a genome.

13. A genome library prepared by the method according to any of claims 1-12.
